# EUROPÄISCHE PATENTSCHRIFT

(11) **EP 3 669 177 B1**
(45) Veröffentlichungstag und Bekanntmachung des Hinweises auf die Patenterteilung: **09.12.2020**
(21) Anmeldenummer: 18796612.2
(22) Anmeldetag: 26.10.2018
(51) Int. Cl.: G01N 21/17, A61B 5/00, G01N 29/24

(54) **PHOTOAKUSTIK-SENSORKOPF UND PHOTOAKUSTIK-MESSAPPARAT MIT VERBESSERTER STÖRSIGNAL-UNTERDRÜCKUNG**
PHOTOACOUSTIC SENSOR HEAD AND PHOTOACOUSTIC MEASURING APPARATUS WITH IMPROVED INTERFERENCE SIGNAL SUPPRESSION
TÊTE DE CAPTEUR PHOTOACOUSTIQUE ET APPAREIL DE MESURE PHOTOACOUSTIQUE À SUPPRESSION AMÉLIORÉE DES SIGNAUX PARASITES

(30) Priorität: 27.10.2017 DE 102017219338
(43) Veröffentlichungstag der Anmeldung: 24.06.2020
(73) Patentinhaber: Humboldt-Universität zu Berlin, 10099 Berlin (DE)
(72) Erfinder: VON LILIENFELD-TOAL, Hermann, 63571 Gelnhausen (DE); KISCHKAT, Jan-Ferenc, 10829 Berlin (DE); SUPPLIE, Oliver, 10963 Berlin (DE)
(74) Vertreter: Schmelcher, Thilo
(86) Internationale Anmeldenummer: PCT/EP2018/079397
(87) Internationale Veröffentlichungsnummer: WO 2019/081701

(56) Entgegenhaltungen:
- JP-A- 2017 046 826
- US-A1- 2010 053 618
- US-A1- 2010 094 134
- US-A1- 2011 112 391
- US-A1- 2014 066 743
- US-B2- 9 453 761
- SCHMID T ET AL: "Photoacoustic absorption spectra of biofilms", REVIEW OF SCIENTIFIC INSTRUMENTS, AIP, MELVILLE, NY, US, Bd. 74, Nr. 1, Januar 2003 (2003-01), Seiten 755-757, XP012040373, ISSN: 0034-6748, DOI: 10.1063/1.1512766

## Beschreibung

Die Erfindung betrifft einen Photoakustik-Sensorkopf zur Detektion akustischer Signale, die in einer Probe durch Absorption eines gepulsten Messlichts angeregt werden, umfassend ein für das Messlicht transparentes Kontaktprisma mit einer Probenkontaktfläche, einer der Probenkontaktfläche gegenüberliegend angeordneten Detektionsfläche und einer der Detektionsfläche benachbart angeordneten Lichteintrittsfläche sowie Mittel zum Einstrahlen des Messlichts durch die Lichteintrittsfläche in Richtung auf die Probenkontaktfläche, wobei eine wenigstens einen Schallwandler umfassende Detektionseinrichtung die Detektionsfläche bedeckend angeordnet ist.

Die Erfindung betrifft ferner einen Photoakustik-Messapparat umfassend einen vorgenannten Photoakustik-Sensorkopf, eine Lichtquelle für gepulstes Messlicht, eine Einrichtung zur Zuführung des Messlichts zum Sensorkopf, eine Einrichtung zur Messdatenerfassung der Detektionseinrichtung und eine Einrichtung zur Beleuchtungssteuerung, die die Lichtquelle zum Aussenden von Messlichtpulsen mit vorbestimmter Pulsdauer zu vorbestimmten Zeitpunkten veranlasst

Photoakustik-Messapparate und -Sensoren sind bekannte Mittel zur Detektion und Quantifizierung von Substanzen durch deren charakteristische Lichtabsorption. Zur Untersuchung einer Probe auf eine gesuchte Substanz erfolgt die Beleuchtung der Probe mit einem Messlicht, das eine oder mehrere Lichtwellenlängen aufweist, die als charakteristische Absorptionswellenlängen der Substanz vorbekannt sind. Das Messlicht wird gepulst in die Probe eingestrahlt und dort in Abhängigkeit von der Verteilung der Substanz lokal unterschiedlich stark absorbiert. Die mit der Lichtabsorption eingetragene Energie bewirkt eine Erwärmung und eine thermomechanische Expansion der Probe. Beides löst Relaxationsprozesse aus, die die inhomogen eingetragene Energie über die Probe verteilen, um das Equilibrium wieder herzustellen. Dies geschieht über Wärmediffusion und die Propagation von Druckwellen - Schallwellen - durch die Probe, u.a. hin zur Probenoberfläche. Eine beispielhafte Ausgestaltung ist aus der US Patentanmeldung US 2011/0112391 A1 bekannt.

In der vorliegenden Erfindung wird das in der Probe durch Lichtabsorption erzeugte Schallsignal detektiert und ausgewertet. Hierfür ist ein Photoakustik-Sensorkopf in mechanischem Kontakt mit einer Probenoberfläche vorgesehen. Der Sensorkopf weist eine für diesen Kontakt vorgesehene Probenkontaktfläche auf, die in der Regel als eine ebene Fläche ausgestaltet ist. Der Sensorkopf besteht aus einem starren Vollmaterial, z.B. aus einem Glas oder aus einem ausgehärteten Kunststoff, und kann eine an der Probenkontaktfläche eintreffende, in der Probe erzeugte Schallwelle wenigsten teilweise aufnehmen und durch das Vollmaterial hindurch auf eine der Probenkontaktfläche gegenüberliegende Außenfläche des Sensorkopfes führen, die als Detektionsfläche bezeichnet ist. Auf der Detektionsfläche ist eine Detektionseinrichtung angeordnet, die wenigstens einen Schallwandler umfasst, der die dort eintreffende Schallwelle detektiert. Übliche Schallwandler sind aus einem piezoelektrischen Material gefertigt und wandeln direkt Druckschwankungen in elektrische Signale um.

Photoakustische Messungen dienen der nicht-invasiven und zerstörungsfreien Bestimmung der Zusammensetzung von Fluiden, Gasen und Festkörpern. Sie sind auch gängig in der medizinischen Diagnostik, wobei ein Sensorkopf mit dem menschlichen Körper in Kontakt gebracht wird, sei es im Körperinnern oder auf der Haut. Mögliche Messanwendungen liegen vor allem in der verletzungsfreien Bestimmung von Blutinhaltsstoffen wie beispielsweise Drogen und Alkohol oder auch Blutzucker.

Die üblichen Messgrößen bei einer photoakustischen Messung sind die Amplituden der eintreffenden Schallsignale und die Zeiten ihres Eintreffens. Aus diesen kann die Antwort der Probe auch in Bezug auf Teilvolumia, z.B. Voxel, der Probe rekonstruiert werden, so dass Rückschlüsse auf die Mengenverteilung absorbierender Substanzen möglich sind. Überdies kann das Messlicht aus einer durchstimmbaren Lichtquelle, z.B. einem Laser, stammen und einen Spektralbereich umfassen. Die einzelnen Messlichtpulse können jeweils unterschiedliche Anteile des Spektralbereichs umfassen und somit auch Schallsignale in Abhängigkeit der Wellenlänge auslösen. Dies erlaubt die Analyse mehrerer oder aller Substanzen in einer Probe mit derselben Messung.

Es ist zur Detektion von Substanzen nahe der Probenoberfläche wichtig, die Probenkontaktfläche, an der das in der Probe erzeugte Schallsignal aufgenommen werden soll, in unmittelbarer Nähe der mit Messlicht beleuchteten Probenfläche anzuordnen. Insbesondere kann der Sensorkopf einen für das Messlicht transparenten Vollmaterialkörper, das sogenannte Kontaktprisma, umfassen. Das Kontaktprisma kann weiterhin eine der Probenkontaktfläche gegenüberliegende, für das Messlicht durchlässige Lichteintrittsfläche sowie dort angeordnete Mittel aufweisen, um das Messlicht in das Kontaktprisma in Richtung auf die Probenkontaktfläche einzustrahlen. Solche Mittel können beispielsweise eine mit Glaslot an der Lichteintrittsfläche befestigte optische Faser umfassen. Durch Einstrahlung auf die Probenkontaktfläche durch das Kontaktprisma hindurch kann eine Schallantwort in den unmittelbar unter der Probenkontaktfläche vorliegenden Probenbereichen zuverlässig erzeugt und detektiert werden.

Es ist bekannter Stand der Technik, das Kontaktprisma und die Mittel zum Einstrahlen des Messlichts so auszulegen, dass das Messlicht die gesamte Probenkontaktfläche ausleuchtet. Ebenso ist es gängig, die Detektionseinrichtung umfassend wenigstens einen Schallwandler so auszugestalten, dass sie die gesamte Detektionsfläche des Kontaktprismas bedeckt. Es sollen dadurch alle Schallsignale, die die Detektionsfläche erreichen, erfasst werden. Dabei ist es möglich, aber nicht erforderlich, dass die Detektionseinrichtung auch ein Array von Schallwandlern umfasst, die nebeneinander auf der Detektionsfläche angeordnet sind.

Bei einer photoakustischen Messung nach dem Stand der Technik wird eine Vielzahl von Messlichtpulsen in die Probe eingestrahlt, und die elektrischen Signale der Detektionseinrichtung werden selektiv mit einem Lock-In-Verstärker verstärkt, der auf die Wiederholrate der Messlichtpulse abgestimmt ist, um das Signal-Rausch-Verhältnis (SNR) zu verbessern.

Signalanteile, die nicht aus der Probe stammen, aber genau im Wiederholtakt der Messlichtpulse auftreten, können aber auf diese Weise nicht wirksam unterdrückt werden. Solche Signalanteile ergeben sich in erster Linie aus der Reflektion eines Anteils des Messlichts an der Probenkontaktfläche, woraufhin dieser Messlichtanteil an inneren Flächen des Kontaktprismas und an Strukturelementen des Sensorkopfes - einschließlich der Detektionseinrichtung - teils reflektiert und teil absorbiert wird, was störende Schalleinträge von mehreren Stellen des Sensorkopfes nach sich ziehen kann. Dieses Störschallsignal entsteht praktisch unverzüglich beim Einstrahlen eines Messlichtpulses, weist aber - insbesondere auch bei Schallreflektionen innerhalb des Kontaktprismas - ein breites Intervall von Schalllaufzeiten auf, bis es zur Detektionseinrichtung gelangt, wo es dann mit dem Nutzschallsignal der Probe überlagert detektiert wird.

Das apparativ bedingte Störsignal steht in derselben zeitlichen Beziehung zu den Messlichtpulsen wie das Nutzsignal und kann deshalb nicht durch Lock-In-Verstärkung beseitigt werden.

Das Patent US 9,453,761 B2 von Ida beschreibt einen Ansatz, apparativ bedingte Störsignale zeitlich von Nutzsignale zu separieren durch eine entsprechende Ausgestaltung des Sensorkopfes. Hierfür sieht Ida vor, dass die Detektionseinrichtung in einem größeren Abstand zur Probe angeordnet wird als das Austrittsende einer Faser, aus der das Messlicht in den Sensorkopf eintritt. Zudem soll ein für das Messlicht durchlässiger Abstandshalter ("arrangement member") zwischen der Austrittsfaser und der Probe angeordnet sein. Durch diese Maßnahmen lässt sich laut Ida ein Anteil des Störsignals ("Noise B") in ein Zeitfenster verschieben, das erst nach dem Ende der Aufzeichnung des Nutzsignals beginnt.

Dabei äußert sich die US 9,453,761 B2 kaum zu den möglichen Quellen der Störsignale. In Spalte 4 ab Zeile 25 der Druckschrift wird behauptet, dass eine photoakustische Welle - eine Schallwelle - in der Nähe des Faseraustrittsendes generiert wird, durch Reflektion an der Probenkontaktfläche dann zur Detektionseinrichtung gelangt und dort schließlich Noise B verursacht. Diese Erklärung überrascht, weil man gewöhnlich das Material des Kontaktprismas und das Messlicht gerade so aufeinander abstimmt, dass es möglichst nicht zur Absorption nennenswerter Energieanteile während der Lichtpropagation im Kontaktprisma kommt.

Die Erfindung stellt sich in einem ersten Aspekt die Aufgabe, einen verbesserten Photoakustik-Sensorkopf vorzuschlagen.

Die Erfindung stellt sich in einem zweiten Aspekt die Aufgabe, einen Photoakustik-Messapparat mit verbesserter Störsignal-Unterdrückung unter Verwendung des verbesserten Photoakustik-Sensorkopfes vorzuschlagen.

Die erste Aufgabe wird gelöst durch einen Photoakustik-Sensorkopf zur Detektion akustischer Signale, die in einer Probe durch Absorption eines gepulsten Messlichts angeregt werden, umfassend ein für das Messlicht transparentes Kontaktprisma mit einer Probenkontaktfläche, einer der Probenkontaktfläche gegenüberliegend angeordneten Detektionsfläche und einer der Detektionsfläche benachbart angeordneten Lichteintrittsfläche sowie Mittel zum Einstrahlen des Messlichts durch die Lichteintrittsfläche in Richtung auf die Probenkontaktfläche, wobei eine wenigstens einen Schallwandler umfassende Detektionseinrichtung die Detektionsfläche bedeckend angeordnet ist, dadurch gekennzeichnet, dass die an der Probenkontaktfläche reflektierten Anteile des Messlichts auf die Detektionsfläche gerichtet sind, oder ein erster Teil des an Probenkontaktfläche reflektierenden Anteiles des Messlichts auf die Detektionsfläche und ein zweiter Teil des an der Probenkontaktfläche reflektierenden Anteiles des Messlichts auf die Lichteintrittsfläche gerichtet ist, wobei zwischen der Detektionsfläche und der Detektionseinrichtung eine Materialschicht enthaltend ein das Messlicht absorbierendes Material angeordnet ist.

Die zweite Aufgabe wird gelöst durch einen Photoakustik-Messapparat umfassend einen erfindungsgemäßen Photoakustik-Sensorkopf, eine Lichtquelle für gepulstes Messlicht, eine Einrichtung zur Zuführung des Messlichts zum Sensorkopf, eine Einrichtung zur Messdatenerfassung der Detektionseinrichtung und eine Einrichtung zur Beleuchtungssteuerung, die die Lichtquelle zum Aussenden von Messlichtpulsen mit vorbestimmter Pulsdauer zu vorbestimmten Zeitpunkten veranlasst, dadurch gekennzeichnet, dass der Messapparat eine mit der Einrichtung zur Beleuchtungssteuerung und der Einrichtung zur Messdatenerfassung in Kommunikation stehende Einrichtung zur Messdatenauswertung umfasst, die die Einrichtung zur Messdatenerfassung veranlasst, die Messdaten der Detektionseinrichtung nur während einer Mehrzahl von nicht überlappenden Zeitintervallen zu erfassen, deren zeitliche Lagen bezogen auf die Zeitpunkte des Aussendens der Messlichtpulse vorbestimmt sind und deren Intervalllängen in Summe kleiner als der zeitliche Abstand zwischen zwei aufeinanderfolgenden Messlichtpulsen sind.

Die Unteransprüche 2 bis 5 geben vorteilhafte Ausgestaltungen des Sensorkopfes an. Die Unteransprüche 7 bis 9 sind auf vorteilhafte Ausgestaltungen des Messapparates gerichtet.

Die Erfindung greift den Ansatz von Ida auf, die Störsignale zeitlich von den Nutzsignalen zu separieren. In den Nutzsignalen treten sie dann nicht mehr auf - sind also dort unterdrückt.

Ausgehend von den eingangs erläuterten Ursachen für das Auftreten der apparativ bedingten Störsignale geht die Erfindung den Weg, den Anteil des Messlichts, der unmittelbar nach Einstrahlung in das Kontaktprisma auf die Probenkontaktfläche trifft und dort in das Kontaktprisma reflektiert wird, möglichst schnell und vollständig aus dem System zu entfernen.

Zu diesem Zweck wird erfindungsgemäß vorgesehen, das Kontaktprisma hinsichtlich seiner Form und der Anordnung seiner Seitenflächen zueinander so auszugestalten, dass die an der Probenkontaktfläche reflektierten Anteile des Messlichts auf die Detektionsfläche gerichtet sind oder ein erster Teil des an Probenkontaktfläche reflektierenden Anteiles des Messlichts auf die Detektionsfläche und ein zweiter Teil des an der Probenkontaktfläche reflektierenden Anteiles des Messlichts auf die Lichteintrittsfläche gerichtet ist, d.h. die einmal reflektierten Lichtstrahlen erreichen ohne weitere Reflektionen an inneren Grenzflächen und ohne das Kontaktprisma zu verlassen die Detektionsfläche oder die Lichteintrittsfläche des Kontaktprismas.

Die Detektionsfläche ist von der Detektionseinrichtung bedeckt, d.h. die Detektionseinrichtung erstreckt sich über die gesamte Detektionsfläche, und es ist erfindungsgemäß vorgesehen, dass zwischen der Detektionsfläche und der Detektionseinrichtung eine Materialschicht enthaltend ein das Messlicht absorbierendes Material angeordnet ist.

Anteile des Messlichts, die auf die Detektionsfläche treffen, werden erfindungsgemäß möglichst vollständig absorbiert, erwärmen die Materialschicht und lösen ein Drucksignal aus, das unverzüglich von der Detektionseinheit erfasst wird. Das so erzeugte Schallsignal ist ein Störsignal relativ großer Amplitude, das nur in einem kurzen Zeitintervall unmittelbar nach Auslösen des Messlichtpulses auftritt. Nutzsignale durch Absorption des Messlichts in der Probe müssen hingegen als Schallwellen zunächst zur Probenkontaktfläche gelangen, in das Kontaktprisma eintreten und dieses bis zur Detektionsfläche durchqueren. Sie treffen dementsprechend mit einer Zeitverzögerung der Größenordnung Mikrosekunden später ein. Das Störsignal wird durch die erfindungsgemäß forcierte Absorption in der Materialschicht zwischen Detektionsfläche und Detektionseinrichtung zeitlich konzentriert und separiert von Nutzsignalen aus der Probe erfasst.

Soweit Anteile des Messlichts existieren, die auf die Lichteintrittsfläche gelangen, verlassen diese dort das Kontaktprisma. Auch in diesem Fall ist das primär an der Probenkontaktfläche reflektierte Licht aus dem Messsystem entfernt, so dass das zeitlich spätere Auftreten von Störsignalen unterdrückt ist.

Bei der Ausgestaltung eines erfindungsgemäßen Sensorkopfs hat der Fachmann die Freiheit der Festlegung von Form und Größe der Probenkontaktfläche und der Art und Weise, wie er diese mit Messlicht zu beleuchten wünscht. Ausgehend von seinen Festlegungen bestimmen sich dann nach der Lehre dieser Erfindung Form und Größe der Detektionsfläche - und damit zugleich der Detektionseinrichtung und der das Messlicht absorbierenden Materialschicht - anhand der für den Fachmann sehr einfachen Beachtung des Reflexionsgesetzes.

In einer bevorzugten Ausgestaltung des Sensorkopfes ist die Materialschicht zwischen Detektionsfläche und Detektionseinrichtung ein Klebstoff zur Befestigung der Detektionseinrichtung am Kontaktprisma. Weiterhin bevorzugt ist, dass das Messlicht absorbierende Material ein Lichtabsorber-Pigment, besonders bevorzugt Carbon Black, ist. Das Pigment kann vorteilhafterweise einem herkömmlichen Klebstoff gesondert beigemengt werden.

Es wird weiterhin als vorteilhaft angesehen, dass die Mittel zum Einstrahlen des Messlichts wenigstens eine Lichtleiterfaser in für das Messlicht optisch transparenter Verbindung mit der Lichteintrittsfläche des Kontaktprismas umfassen, wobei das aus dem Faserende austretende und hiernach auffächernde Messlicht die gesamte Probenkontaktfläche ausleuchtet. Es kann von Vorteil sein, eine Mehrzahl von Lichtleiterfaser nebeneinander in einer linearen Anordnung zum Einstrahlen des Messlichts in das Kontaktprisma vorzusehen. Auf diese Weise kann eine sich zum Verlauf der linearen Anordnung parallel erstreckende Probenkontaktfläche gleichmäßig ausgeleuchtet werden.

Ferner ist es eine bevorzugte Ausgestaltung des Photoakustik-Sensorkopfes, dass auf dem wenigstens einen Schallwandler der Detektionseinrichtung ein Backing-Material angeordnet ist, dessen akustische Impedanz größer ist als die des Schallwandlermaterials.

Nachfolgend werden Beispiele zur Ausgestaltung des Photoakustik-Sensorkopfes näher erläutert, auch anhand von Figuren. Dabei zeigt:
Fig. 1 mögliche Ausgestaltungen des Photoakustik-Sensorkopfes in Seitenansichten a) und b) und in Aufsichten c) und d), respektive;
Fig. 2 einen schematischen Plot der gemessenen Druckamplituden des Störsignals (gestrichelt) und des Nutzsignals der Probe in der erzeugten zeitlichen Separation.

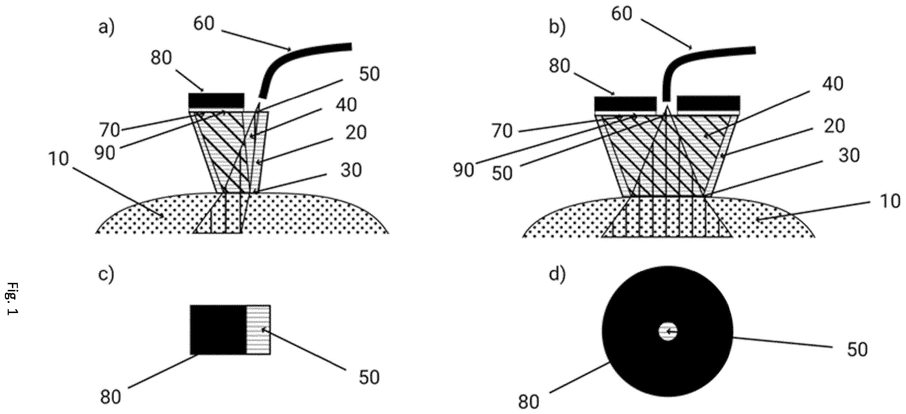

In Fig. 1 sind zwei mögliche Ausgestaltungen des erfindungsgemäßen Photoakustik-Sensorkopfes dargestellt. Die Teilfiguren 1 a) und b) zeigen jeweils eine Probe 10, auf die ein Kontaktprisma 20 angeordnet wird derart, dass sich Probe 10 und Kontaktprisma 20 an der Probenkontaktfläche 30 berühren. Das Material des Kontaktprismas 20 ist transparent für das Messlicht 40, das über wenigstens eine Lichtleiterfaser 60 durch die Lichteintrittsfläche 50 in das Kontaktprisma 20 in Richtung auf die Probenkontaktfläche 30 eingestrahlt wird.

Üblicherweise weist das Messlicht 40 Wellenlängen aus dem Infrarot-Spektrum, insbesondere aus dem nahen (NIR) und mittleren Infrarot (MIR). Das Messlicht 40 kann für manche Zwecke aber auch sichtbares Licht (VIS) sein oder aus einem anderen nicht-ionisierenden Spektralbereich stammen.

Die Wahl des Materials des Kontaktprismas 20 orientiert sich durch die Transparenzanforderung an den Wellenlängen des Messlichts 40. Beispielsweise für Licht im mittleren infraroten Spektrum (MIR) sind halbleitende Materialien wie Germanium, Zinkselenid, Silizium, Indiumphosphid, Galliumarsenid oder chalkogenide Gläser geeignet, und für Licht im nahen infraroten Spektrum (NIR) oder sichtbaren Spektrum (VIS) eignen sich Siliziumdioxid (Quarz, Gläser), Aluminiumoxid (Korund, Saphir, Rubin) oder auch manche Kunststoffe (z.B. Polyethylen).

Die Mittel zum Einstrahlen des Messlichts 40 sind in der Teilfiguren 1 a) eine oder mehrere Lichtleiterfasern 60, die an der Lichteintrittsfläche 50 des Kontaktprismas (20) fixiert angeordnet sind. Die Fixierung ist nicht dargestellt. Das aus den Fasern 60 austretende Messlicht 40 fächert im Kontaktprisma 20 auf und beleuchtet die gesamte Probenkontaktfläche 30. Ein erster Anteil des Messlichts 40 dringt in die Probe 10 ein und regt Nutzsignale an, während ein zweiter Anteil des Messlichts 40 in Richtung auf die Detektionsfläche 70 reflektiert wird. Die Detektionsfläche 70 ist bedeckt von der Detektionseinrichtung 80, die wenigstens einen Schallwandler umfasst. In der Regel umfasst die Detektionseinrichtung 80 nur einen einzelnen Schallwandler, der sich über die gesamte Detektionsfläche 70 erstreckt. Die Detektionseinrichtung 80 ist beispielsweise auf die Detektionsfläche 70 des Kontaktprismas 20 aufgeklebt mittels der Materialschicht 90, die auch Lichtabsorber-Partikel enthält. Der in der Materialschicht 90 eintreffende zweite Anteil des Messlichts 40 wird möglichst vollständig absorbiert, was ein Störsignal auslöst. Die Detektionseinrichtung 80 erfasst das Störsignal, bevor ein Nutzsignal von der Probe 10 eintreffen kann.

In der Ausgestaltung von Teilfigur 1 b) wird ein kleiner Anteil des Messlichts 40 auch auf die Lichteintrittsfläche 50 und damit in das Austrittsende der Faser 60 zurückgespiegelt. Dieser Fall ist typisch, wenn das Messlicht 40 senkrecht auf die Probenkontaktfläche 30 eingestrahlt wird.

Die Teilfiguren 1 c) und d) zeigen jeweils eine Aufsicht der Sensorköpfe aus den Teilfiguren 1 a) und b), respektive, wobei in Richtung auf die Probe 10 geblickt wird. Die Lichteintrittsfläche 50 in Fig. 1 c) ist rechteckig ausgestaltet, sodass auch eine Mehrzahl von Lichtleiterfasern 60 in einer Linie entlang der langen Rechteckachse angeordnet und fixiert werden kann. In Fig. 1 d) liegt die Lichteintrittsfläche 50 im Zentrum der Detektionsfläche 70 mit der Detektionseinrichtung 80. Auch diese Konfiguration, bei der die Lichteintrittsfläche 50 von der Detektionsfläche 70 umgeben ist, soll als benachbarte Anordnung der Lichteintrittsfläche 50 zur Detektionsfläche 70 verstanden werden.

Ein Photoakustik-Messapparat mit einem erfindungsgemäßen Photoakustik-Sensorkopf kann speziell zur vorteilhaften Verwendung des Sensorkopfes ausgebildet werden, indem eine Messdatenauswerteeinrichtung hinzugefügt wird, die das unverzüglich nach dem Einstrahlen des Messlichts 40 auftretende, isolierte Störsignal angemessen berücksichtigt.

Neben dem Sensorkopf umfasst der Messapparat eine Lichtquelle für gepulstes Messlicht 40, eine Einrichtung zur Zuführung des Messlichts 40 zum Sensorkopf, eine Einrichtung zur Messdatenerfassung der Detektionseinrichtung 80 und eine Einrichtung zur Beleuchtungssteuerung, die die Lichtquelle zum Aussenden von Messlichtpulsen mit vorbestimmter Pulsdauer zu vorbestimmten Zeitpunkten veranlasst. Überdies soll der Messapparat eine mit der Einrichtung zur Beleuchtungssteuerung und der Einrichtung zur Messdatenerfassung in Kommunikation stehende Einrichtung zur Messdatenauswertung umfassen. Die Einrichtung zur Messdatenauswertung veranlasst die Einrichtung zur Messdatenerfassung, die Messdaten der Detektionseinrichtung 80 nur während einer Mehrzahl von nicht überlappenden Zeitintervallen zu erfassen, deren zeitliche Lagen bezogen auf die Zeitpunkte des Aussendens der Messlichtpulse vorbestimmt sind und deren Intervalllängen in Summe kleiner als der zeitliche Abstand zwischen zwei aufeinanderfolgenden Messlichtpulsen sind.

Anders gesagt wird die Zeitspanne zwischen dem Aussenden zweier aufeinanderfolgender Messlichtpulse in nicht-überlappende Zeitintervalle unterteilt, von denen einige, aber nicht alle, zur Messdatenerfassung vorgesehen werden. Die Einrichtung zur Messdatenauswertung gibt die Zeitintervalle mit Datenerfassung nach Vorbestimmung durch den Nutzer vor. Beispielsweise umfasst die Einrichtung zur Messauswertung eine Stoppuhr, die bei Auslösen eines Messlichtpulses zurückgesetzt wird, sowie eine Tabelle mit Stoppuhranzeigen, bei denen Zeitintervalle beginnen und enden, in denen Messdaten erfasst werden sollen. In einer möglichen Ausgestaltung weist die Einrichtung zur Messdatenauswertung die Einrichtung zur Messdatenerfassung an, bei Vorliegen einer tabellierten Stoppuhranzeige die Datenerfassung zu aktivieren oder zu deaktivieren. Die Einrichtung zur Datenerfassung umfasst wenigstens einen nicht-flüchtigen elektronischen Datenspeicher, der die von der Detektionseinrichtung 80 erhaltenen Spannungswerte während der zur Datenerfassung vorbestimmten Zeitintervalle digital speichert.

Vorzugsweise bilden die Einrichtung zur Messdatenerfassung und die Einrichtung zur Messdatenauswertung eine bauliche Einheit. Sie lassen sich besonders einfach im Wege der Programmierung eines herkömmlichen Personal Computers realisieren.

Zur Beseitigung statistischen Rauschens ist es sehr vorteilhaft, die erfassten Messdaten über eine Mehrzahl von Messlichtpulsen, d.h. über mehrere Zeitintervalle mit jeweils demselben zeitlichen Bezug zum Aussenden eines Messlichtpulses, zu mitteln. Vorzugsweise veranlasst dies die Einrichtung zur Messdatenauswertung, indem sie ihre Zeitvorgaben an die Einrichtung zur Messdatenerfassung über eine Sequenz von Messlichtpulsen hinweg wiederholt. Die erfassten Messdaten können dabei nach Art des bekannten Boxcar-Averaging im Datenspeicher der Einrichtung zur Datenerfassung aufaddiert und hiernach durch die Anzahl der Messlichtpulse geteilt werden, um eine Mittelwertbildung durchzuführen.

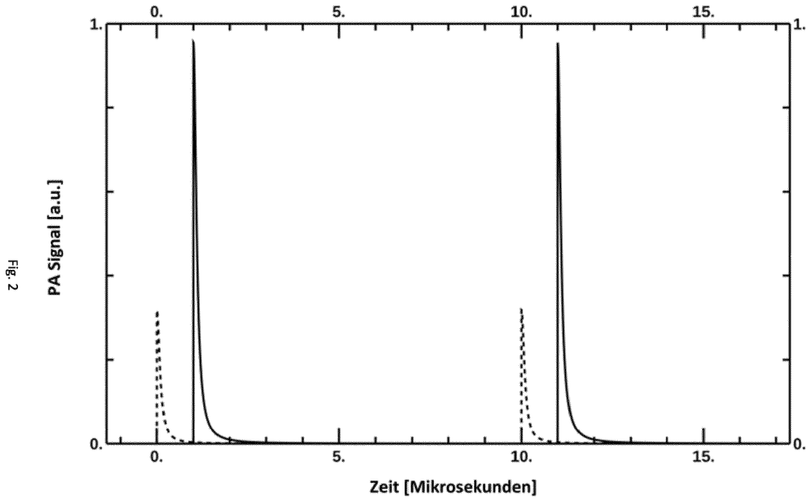

In Fig. 2 ist ein schematischer Plot des zeitlichen Verlaufs der Druckamplitude (PA) für zwei aufeinander folgende Messlichtpulse gezeichnet. Statistisches Rauschen ist dabei nicht berücksichtigt. Die durchgezogenen Kurven repräsentieren das akustische Nutzsignal aus der Probe, das erst durch das Kontaktprisma hindurch propagieren muss, um die Detektionseinrichtung zu erreichen. Es trifft daher deutlich nach dem - gestrichelt dargestellten - Störsignal ein, dass unmittelbar nach dem Aussenden eines Messlichtpulses generiert wird. Beide Signale lassen sich in separaten, nicht-überlappenden Zeitintervallen erfassen. Die Länge des ersten Zeitintervalls (Störsignal) ist dabei apparativ bestimmt und von der Probe unabhängig. Die Länge des zweiten Zeitintervalls (Nutzsignal) kann vom Nutzer vorbestimmt werden; insbesondere kann sie sehr viel größer als die Länge des ersten Zeitintervalls sein. Beide Zeitintervalle zusammen sind kürzer als der Zeitabstand zwischen den Messlichtpulsen.

Es erscheint in vielen Fällen als ausreichend und daher auch vorteilhaft, dass die Mehrzahl von Zeitintervallen nach dem Aussenden eines Messlichtpulses genau zwei Zeitintervalle umfasst. In diesem Fall ist es vorzugsweise vorgesehen, dass das erste Zeitintervall beginnt zum Zeitpunkt des Aussendens des Messlichtpulses und endet, bevor ein in der Probe erzeugtes und durch die Probenkontaktfläche in das Kontaktprisma eintretendes Schallsignal die Detektionsfläche erreicht.

Es erscheint in vielen Fällen als ausreichend und daher auch vorteilhaft, dass die Mehrzahl von Zeitintervallen nach dem Aussenden eines Messlichtpulses genau zwei Zeitintervalle umfasst. In diesem Fall ist es vorzugsweise vorgesehen, dass das erste Zeitintervall beginnt zum Zeitpunkt des Aussendens des Messlichtpulses und endet, bevor ein in der Probe erzeugtes und durch die Probenkontaktfläche in das Kontaktprisma eintretendes Schallsignal die Detektionsfläche erreicht.

## Patentansprüche

1. Photoakustik-Sensorkopf zur Detektion akustischer Signale, die in einer Probe (10) durch Absorption eines gepulsten Messlichts (40) angeregt werden, umfassend ein für das Messlicht (40) transparentes Kontaktprisma (20) mit einer Probenkontaktfläche (30), einer der Probenkontaktfläche (30) gegenüberliegend angeordneten Detektionsfläche (70) und einer der Detektionsfläche (70) benachbart angeordneten Lichteintrittsfläche (50) sowie Mittel (60) zum Einstrahlen des Messlichts (40) durch die Lichteintrittsfläche (50) in Richtung auf die Probenkontaktfläche (30), wobei eine wenigstens einen Schallwandler umfassende Detektionseinrichtung (80) die Detektionsfläche (70) bedeckend angeordnet ist, **dadurch gekennzeichnet, dass** die an der Probenkontaktfläche (30) reflektierten Anteile des Messlichts (40) auf die Detektionsfläche (70) gerichtet sind, oder ein erster Teil des an Probenkontaktfläche (30) reflektierenden Anteiles des Messlichts (40) auf die Detektionsfläche (70) und ein zweiter Teil des an der Probenkontaktfläche (30) reflektierenden Anteiles des Messlichts (40) auf die Lichteintrittsfläche (50) gerichtet ist, wobei zwischen der Detektionsfläche (70) und der Detektionseinrichtung (80) eine Materialschicht (90) enthaltend ein das Messlicht (40) absorbierendes Material angeordnet ist.

2. Photoakustik-Sensorkopf nach Anspruch 1, **dadurch gekennzeichnet, dass** die zwischen Detektionsfläche (70) und Detektionseinrichtung (80) angeordnete Materialschicht (90) ein Klebstoff zur Befestigung der Detektionseinrichtung (80) am Kontaktprisma (20) ist.

3. Photoakustik-Sensorkopf nach einem der vorangehenden Ansprüche, **dadurch gekennzeichnet, dass** das das Messlicht (40) absorbierende Material ein Lichtabsorber-Pigment, vorzugsweise Carbon Black, ist.

4. Photoakustik-Sensorkopf nach einem der vorangehenden Ansprüche, **dadurch gekennzeichnet, dass** die Mittel (60) zum Einstrahlen des Messlichts (40) wenigstens eine Lichtleiterfaser in für das Messlicht (40) optisch transparenter Verbindung mit der Lichteintrittsfläche (50) des Kontaktprismas (20) umfassen, wobei das aus dem Faserende austretende und hiernach auffächernde Messlicht (40) die gesamte Probenkontaktfläche (30) ausleuchtet.

5. Photoakustik-Sensorkopf nach Anspruch 4, **dadurch gekennzeichnet, dass** die Mittel (60) zum Einstrahlen des Messlichts (40) eine Mehrzahl von Lichtleiterfasern nebeneinander in einer linearen Anordnung umfassen.

6. Photoakustik-Sensorkopf nach einem der vorangehenden Ansprüche, **dadurch gekennzeichnet, dass** auf dem wenigstens einen Schallwandler der Detektionseinrichtung (80) ein Backing-Material angeordnet ist, dessen akustische Impedanz größer ist als die des Schallwandlermaterials.

7. Photoakustik-Messapparat umfassend einen Photoakustik-Sensorkopf nach einem der vorangehenden Ansprüche, eine Lichtquelle für gepulstes Messlicht (40), eine Einrichtung zur Zuführung des Messlichts (40) zum Sensorkopf, eine Einrichtung zur Messdatenerfassung der Detektionseinrichtung (80) und eine Einrichtung zur Beleuchtungssteuerung, die die Lichtquelle zum Aussenden von Messlichtpulsen (40) mit vorbestimmter Pulsdauer zu vorbestimmten Zeitpunkten veranlasst, **dadurch gekennzeichnet, dass** der Messapparat eine mit der Einrichtung zur Beleuchtungssteuerung und der Einrichtung zur Messdatenerfassung in Kommunikation stehende Einrichtung zur Messdatenauswertung umfasst, die die Einrichtung zur Messdatenerfassung veranlasst, die Messdaten der Detektionseinrichtung (80) nur während einer Mehrzahl von nicht überlappenden Zeitintervallen zu erfassen, deren zeitliche Lagen bezogen auf die Zeitpunkte des Aussendens der Messlichtpulse (40) vorbestimmt sind und deren Intervalllängen in Summe kleiner als der zeitliche Abstand zwischen zwei aufeinanderfolgenden Messlichtpulsen (40) sind.

8. Photoakustik-Messapparat nach Anspruch 7, **dadurch gekennzeichnet, dass** die Einrichtung zur Messdatenauswertung das Mitteln der erfassten Messdaten über eine Mehrzahl von Messlichtpulsen (40) veranlasst oder durchführt.

9. Photoakustik-Messapparat nach einem der Ansprüche 7 oder 8, **dadurch gekennzeichnet, dass** die Mehrzahl von Zeitintervallen nach dem Aussenden eines Messlichtpulses (40) genau zwei Zeitintervalle umfasst.

10. Photoakustik-Messapparat nach Anspruch 8, **dadurch gekennzeichnet, dass** das erste Zeitintervall beginnt zum Zeitpunkt des Aussendens des Messlichtpulses (40) und endet, bevor ein in der Probe (10) erzeugtes und durch die Probenkontaktfläche (30) in das Kontaktprisma (20) eintretendes Schallsignal die Detektionsfläche (70) erreicht.

## Claims

1. A photo-acoustic sensor head for the detection of acoustic signals, which are triggered in a sample (10) through absorption of a pulsed measuring light (40), comprising a contact prism (20) transparent for the measuring light (40) with a sample contact surface (30), a detection surface (70) arranged opposite the sample contact surface (30) and a light entry surface (50) arranged adjacently to the detection surface (70) as well as means (60) for irradiating the measuring light (40) through the light entry surface (50) in direction of the sample contact surface (30), wherein a detection device (80) comprising at least one sound converter is arranged so as to cover the detection surface (70), **characterized in that** the portions of measuring light (40) reflected at the sample contact surface (30) are directed at the detection surface (70) or a first portion of the reflected portion of measuring light reflected at the sample contact surface (30) is directed at the detection surface (70) and a second portion of the reflected portion of measuring light reflected at the sample contact surface (30) is directed at the light entry surface (50), wherein a material layer (90) containing a material absorbing the measuring light (40) is arranged between the detection surface (70) and the detection device (80).

2. Photo-acoustic sensor head according to claim 1, **characterized in that** the material layer (90) arranged between the detection surface (70) and the detection device (80) is an adhesive for fastening the detection device (80) to the contact prism (20).

3. The photo-acoustic sensor head according to one of the preceding claims, **characterized in that** the material absorbing the measuring light (40) is a light absorber pigment, preferably carbon black.

4. Photo- -acoustic sensor head according to one of the preceding claims, **characterized in that** the means (60) for irradiating the measuring light (40) comprise at -least one optical fiber connected in a manner optically transparent manner for the measuring light (40) to the light entry surface (50) of the contact prism (20), wherein the measuring light (40) exiting from the fiber end and thereupon fanning out illuminates the entire sample contact surface (30).

5. Photo- -acoustic sensor head according to claim 4, **characterized in that** the means (60) for irradiating the measuring light (40) comprise a plurality of optical fibers adjacent to each other in a linear arrangement.

6. Photo-acoustic sensor head according to one of the preceding claims, **characterized in that** a backing material is arranged on the at least one sound converter of the detection device (80), the acoustic impedance of which is greater than that of the sound converter material.

7. A photo-acoustic measuring apparatus comprising a photo-acoustic sensor head according to one of the preceding claims, a light source for pulsed measuring light (40), a device for supplying measuring light (40) to the sensor head, a device for measured data recording of the detection device (80) and a device for lighting control, which causes the light source to emit measuring light pulses (40) of a predetermined pulse duration at predetermined points of time, **characterized in that** the measuring apparatus comprises a device for measured data evaluation communicating with the device for lighting control and with the device for measured data recording, which causes the device for measured data recording to record the measured data of the detection device (80) only during a plurality of non-overlapping time intervals, the temporal positions of which are -predetermined as regards the points in time of emitting the measuring light pulses (40), and the interval lengths of which in total are smaller than the temporal distance between two successive measuring light pulses (40).

8. Photo-acoustic -acoustic measuring apparatus according to claim 7, **characterized in that** the device for measured data evaluation causes the recorded measured data to be averaged across a plurality of measuring light pulses (40) or performs this averaging.

9. Photo-acoustic measuring apparatus according to one of claims 7 or 8, **characterized in that** the plurality of time intervals after emitting a measuring light pulse (40) comprises exactly two time intervals.

10. Photo-acoustic -acoustic measuring apparatus according to claim 8, **characterized in that** the first time interval starts at the point in time of emitting the measuring light pulse (40) and ends before an acoustic signal generated in the sample (10) and entering into the contact prism (20) through the sample contact surface (30) reaches the detection surface (70).

## Revendications

1. Tête de capteur photoacoustique pour la détection de signaux acoustiques, qui sont activés dans un échantillon (10) par absorption d'une lumière de mesure pulsée (40), comprenant un prisme de contact (20) transparent pour la lumière de mesure (40) avec une surface de contact d'échantillon (30), une surface de détection (70) disposée opposée à la surface de contact d'échantillon (30) et une surface d'entrée de lumière (50) disposée à proximité de la surface de détection (70) ainsi que des moyens (60) pour projeter la lumière de mesure (40) à travers la surface d'entrée de lumière (50) en direction de la surface de contact d'échantillon (30), sachant qu'un système de détection (80) comprenant au moins un transducteur acoustique est disposé recouvrant la surface de détection (70), **caractérisée en ce que** les portions de lumière de mesure (40) réfléchissant sur la surface de contact d'échantillon (30) sont dirigées sur la surface de détection (70) ou une première partie de la portion de lumière de mesure (40) réfléchissant sur la surface de contact d'échantillon (30) est dirigée sur la surface de détection (70) et une deuxième partie de la portion de lumière de mesure (40) réfléchissant sur la surface de contact d'échantillon (30) est dirigée sur la surface d'entrée de lumière (50), sachant qu'une couche de matériau (90) contenant un matériau absorbant la lumière de mesure (40) est disposée entre la surface de détection (70) et le système de détection (80).

2. Tête de capteur photoacoustique selon la revendication 1, **caractérisée en ce que** la couche de matériau (90) disposée entre la surface de détection (70) et le système de détection (80) est un agent adhésif pour fixer le système de détection (80) sur le prisme de contact (20).

3. Tête de capteur photoacoustique selon l'une quelconque des revendications précédentes, **caractérisée en ce que** le matériau absorbant la lumière de mesure (40) est un pigment absorbant la lumière, de préférence du noir de carbone.

4. Tête de capteur photoacoustique selon l'une quelconque des revendications précédentes, **caractérisée en ce que** les moyens (60) de projection de la lumière de mesure (40) comprennent au moins une fibre optique en liaison optiquement transparente pour la lumière de mesure (40) avec la surface d'entrée de lumière (50) du prisme de contact (20), sachant que la lumière de mesure (40) sortant de l'extrémité de la fibre et s'étalant après cela éclaire toute la surface de contact d'échantillon (30).

5. Tête de capteur photoacoustique selon la revendication 4, **caractérisée en ce que** les moyens (60) de projection de la lumière de mesure (40) comprennent une pluralité de fibres optiques les unes à côté des autres dans une disposition linéaire.

6. Tête de capteur photoacoustique selon l'une quelconque des revendications précédentes, **caractérisée en ce qu'**un matériau de support est disposé sur au moins un transducteur acoustique du système de détection (80) dont l'impédance acoustique est supérieure à celle du matériau de transducteur acoustique.

7. Appareil de mesure photoacoustique comprenant une tête de capteur photoacoustique selon l'une quelconque des revendications précédentes, une source de lumière pour lumière de mesure pulsée (40), un système pour acheminer la lumière de mesure (40) à la tête de capteur, un système pour la saisie des données de mesure du système de détection (80) et un système de commande d'éclairage, qui déclenche la source de lumière pour émettre des impulsions de lumière de mesure (40) avec une durée d'impulsion prédéterminée à des moments prédéfinis, **caractérisé en ce que** l'appareil de mesure comprend un système d'exploitation des données de mesure en communication avec le système de commande d'éclairage et le système de saisie de données de mesure, qui déclenche le système pour la saisie de données de mesure à saisir les données de mesure du système de détection (80) uniquement pendant une pluralité d'intervalles de temps ne se chevauchant pas, dont les situations en fonction du temps sont prédéterminées en se référant aux moments de l'émission des impulsions de lumière de mesure (40) et dont les longueurs d'intervalle sont au total plus petites que l'intervalle de temps entre deux impulsions de lumière de mesure (40) successives.

8. Appareil de mesure photoacoustique selon la revendication 7, **caractérisé en ce que** le système d'exploitation des données de mesure déclenche ou effectue la moyenne des données de mesure acquises sur une pluralité d'impulsions de lumière de mesure (40).

9. Appareil de mesure photoacoustique selon l'une quelconque des revendications 7 ou 8, **caractérisé en ce que** la pluralité d'intervalles de temps comprend exactement deux intervalles de temps après l'émission d'une impulsion de lumière de mesure (40).

10. Appareil de mesure photoacoustique selon la revendication 8, **caractérisé en ce que** le premier intervalle de temps commence au moment de l'émission de l'impulsion de lumière de mesure (40) et se termine avant qu'un signal sonore produit dans l'échantillon (10) et entrant par la surface de contact d'échantillon (30) dans le prisme de contact (20) n'atteigne la surface de détection (70).
